# EUROPEAN PATENT SPECIFICATION

(11) **EP 3 390 332 B1**
(45) Date of publication and mention of the grant of the patent: **18.05.2022**
(21) Application number: 16822143.0
(22) Date of filing: 15.12.2016
(51) Int. Cl.: C07C 29/149, C07C 29/141, C07C 31/20, B01J 23/63, B01J 23/46

(54) **HYDROGENATION OR HYDROGENOLYSIS OF AN OXYGENATE**
HYDRIERUNG ODER HYDROGENOLYSE EINES OXYGENATS
HYDROGÉNATION OU HYDROGÉNOLYSE D'UN COMPOSÉ OXYGÉNÉ

(30) Priority: 17.12.2015 US 201562268604 P
(43) Date of publication of application: 24.10.2018
(73) Proprietor: Shell Internationale Research Maatschappij B.V., 2596 HR Den Haag (NL)
(72) Inventor: DE VLIEGER, Dionysius, Jacobus, Maria, 1031HW Amsterdam (NL); LANGE, Jean Paul, Andre, Marie, Joseph, Ghislain, 1031 HW Amsterdam (NL); EDULJI, Smita, Houston, Texas 77082 (US); COLIJN, Hendrik, Albertus, 1031 HW Amsterdam (NL)
(74) Representative: Shell Legal Services IP
(86) International application number: PCT/EP2016/081294
(87) International publication number: WO 2017/103009

(56) References cited:
- WO-A1-01/17677
- WO-A1-2015/027184
- WO-A2-02/090601
- US-A1- 2002 133 048
- US-A1- 2013 184 495
- US-A1- 2014 171 694
- JEAN-PAUL LANGE ET AL: "VALERIC BIOFUELS: A PLATFORM OF CELLULOSIC TRANSPORTATION FUELS", ANGEWANDTE CHEMIE INTERNATIONAL EDITION, WILEY-VCH VERLAG GMBH, vol. 49, no. 26, 14 June 2010 (2010-06-14) , pages 4479-4483, XP002677265, ISSN: 1433-7851, DOI: 10.1002/ANIE.201000655 [retrieved on 2010-05-05]

## Description

### Field of the Invention

The present invention relates to a process for the hydrogenation or hydrogenolysis of an oxygenate in the presence of a catalyst, hydrogen and liquid water.

### Background of the Invention

Ethylene glycol and propylene glycol are valuable materials with a multitude of commercial applications, e.g. as heat transfer media, antifreeze, and precursors to polymers, such as PET. Ethylene and propylene glycols are typically made on an industrial scale by hydrolysis of the corresponding alkylene oxides, which are the oxidation products of ethylene and propylene, produced from fossil fuels.

In recent years, increased efforts have focused on producing chemicals, including glycols, from renewable feedstocks, such as sugar-based materials. The conversion of sugars to glycols can be seen as an efficient use of the starting materials with the oxygen atoms remaining intact in the desired product.

Current methods for the conversion of saccharides to sugars revolve around a hydrogenation/hydrogenolysis process as described in Angew. Chem. Int. Ed. 2008, 47, 8510-8513. Sponge metal catalysts such as Raney nickel are often used as the hydrogenation catalyst. Small amounts of leaching may occur with these catalysts and such leaching can lead to the presence of metal in the product or could lead to catalyst deactivation.

WO2015028398 describes a continuous process for the conversion of a saccharide-containing feedstock into glycols. In this process the saccharide-containing feedstock is contacted in a reactor with a catalyst, hydrogen and a solvent. For example, a solution of glucose in water is contacted with a W/Ni/Pt on zirconia catalyst and a Ru on silica catalyst in the presence of hydrogen. The temperature of the reaction is 195°C and the absolute pressure is 75 bar.

The present inventors have observed that in the hot aqueous conditions used for the production of glycols from saccharide-containing feedstocks, many commonly-used inorganic oxide catalyst supports are not stable. For example, the catalyst supports may undergo phase changes or growth of crystallites, or may begin to dissolve. This can detrimentally affect catalyst performance, leading to lower glycol yield and a need to change the catalyst more frequently. This can also lead to system instability such that reaction conditions may need to be changed to maintain catalyst performance. Additionally, dissolution of catalyst supports can lead to the presence of impurities in the glycol process.

Carbon catalyst supports might potentially be stable under the hot, aqueous conditions but may also be mechanically fragile such that a portion of the catalyst is crushed when the supported catalyst is loaded into a reactor. Additionally, carbonaceous deposits may form on the catalysts during the glycol production process, and a typical regeneration procedure of burning off the carbonaceous deposits would not be possible with a carbon catalyst support as the carbon support would also burn.

Similar problems are likely to occur in other processes wherein hydrogenation and hydrogenolysis takes place in the presence of hydrogen and liquid water. Such processes could include hydrogenation of maleic anhydride or succinic acid to tetrahydrofuran and 1,4-butanediol; hydrogenation of levulinic acid to gamma valerolactone; hydrogenation of various monoacids to the corresponding monoalcohols (acetic acid to ethanol); and conversion of carbohydrates or sugar alcohols to hydrogen and carbon dioxide via the aqueous phase reforming reaction.

Duan et al in Catalysis Today 234 (2014), 66-74 discuss the use of titania and zirconia catalyst supports in the aqueous-phase hydrodeoxygenation of sorbitol. The addition of silica to titania improved the hydrothermal stability but did not inhibit the crystallisation and decrease of surface area under the hydrothermal conditions.

Dias et al. (WO2015/027184) discloses a process for the conversion of adipic acid to 1,6-hexanediol by reacting adipic acid with hydrogen in the presence of a heterogeneous catalyst including a first metal (Pt and/or Rh) and a second metal (Mo, W, and/or Re) on a Al₂O₃, IiO₂, ZrO₂, SiO₂, zeolite, and/or carbon support.

In addition, Dias et al. (US2013/184495) discloses a process for preparing hexamethylenediamine by converting glucose and/or fructose to furfural and reacting furfural in the presence of a heterogeneous reduction catalyst comprising Mo, La, SM, Y, W, or Re on a support selected from the group consisting of zirconias, silicas, and zeolites.

Powell et al. (US2014/171694) describes a water stable hydrogenolysis catalyst including (a) sulphur, (b) Mo or W, and (c) Co and/or Ni, incorporated into a Group 4 metal oxide support.

In US2002/133048, Elliott et al. disclose a method for converting sugars to sugar alcohols by passing an aqueous sugar solution over a catalyst comprising ruthenium on a titania support. Related WO2001/17677, by Elliott et al., relates to catalysts that have an active metal on a titania support and methods that use those catalysts in the hydrogenation of organic compounds in the aqueous phase. Finally, in WO2002/090601, Elliott et al. describe a lactose conversion method involving a hydrogenation catalyst that includes ruthenium disposed on a titania support.

Lange et al. ("Valeric Biofuels: A Platform of Cellulosic Transportation Fuels" Angew. Chem. Int. Ed. 49:26:4479-4483; 2010) discloses catalysts with platinum on titania or zirconia for hydrogenating levulinic acid to γ-valerolactone.

The present inventors have sought to provide a hydrogenation or hydrogenolysis process, suitable for use in the hydrogenation of glycolaldehyde during the production of glycols from saccharide-containing feedstocks, wherein the problems of the prior art are avoided.

### Summary of the Invention

Accordingly, the present invention provides a process for the hydrogenation or hydrogenolysis of an oxygenate in a reactor in the presence of a catalyst, hydrogen and liquid water, wherein the catalyst comprises one or more elements from Groups 8 to 11 of the periodic table dispersed on an oxide support, and wherein the oxide support is a mixed metal oxide comprising at least 20wt% titania and at least 20wt% zirconia and at least 80wt% of the mixed metal oxide is made up of titania and zirconia. The present inventors have found that if the catalyst support is a titania-zirconia mixed metal oxide then it is possible to provide catalysts that are stable under the reaction conditions, thereby providing an improved hydrogenation or hydrogenolysis process that avoids the problems associated with thermally unstable catalyst supports.

### Detailed Description of the Invention

The present invention provides a process for the hydrogenation or hydrogenolysis of an oxygenate in a reactor in the presence of a catalyst, hydrogen and liquid water. In a hydrogenation reaction, hydrogen is added to a double or triple bond in a molecule. In a hydrogenolysis reaction, hydrogen cleaves a bond in a molecule. Oxygenates are organic compounds that contain oxygen such as alcohols, ethers, aldehydes and ketones. Preferably the oxygenate is present in or derived from a saccharide-containing feedstock, and the process of the invention produces glycols. The saccharide-containing feedstock preferably comprises starch and/or compounds prepared by the hydrolysis of starch. Glucose may be prepared by the hydrolysis of starch or other methods and is another preferred component of the saccharide-containing feedstock. The saccharide-containing feedstock may also comprise one or more further saccharides selected from the group consisting of monosaccharides other than glucose, disaccharides, oligosaccharides and polysaccharides other than starch. Examples of polysaccharides other than starch include cellulose, hemicelluloses, glycogen, chitin and mixtures thereof.

The saccharide-containing feedstock may be derived from grains such as corn, wheat, millet, oats, rye, sorghum, barley or buckwheat, from rice, from pulses such as soybean, pea, chickpea or lentil, from bananas and/or from root vegetables such as potato, yam, sweet potato, cassava and sugar beet, or any combinations thereof. A preferred source of saccharide-containing feedstock is corn.

A glycols product stream is preferably produced in the process of the invention. Typically this is a mixture of glycols, wherein the main constituents are monoethylene glycol (MEG), monopropylene glycol (MPG) and 1,2-butanediol (1,2-BDO).

The process takes place in the presence of liquid water. The oxygenate is preferably supplied as an aqueous solution of the oxygenate in water.

The process takes place in the presence of hydrogen. Preferably, the process takes place in the absence of air or oxygen. In order to achieve this in a batch process, it is preferable that the atmosphere in the reactor be evacuated and replaced an inert gas, such as nitrogen, and then with hydrogen repeatedly, after loading of any initial reactor contents, before the reaction starts. In order to achieve this in a continuous process, it is preferable that any inert gas is flushed out by maintaining hydrogen flow for a sufficient time.

The process takes place in the presence of a catalyst, wherein the catalyst comprises one or more elements from Groups 8 to 11 of the periodic table dispersed on an oxide support. Preferably the catalyst comprises one or more elements from the group consisting of iron, cobalt, nickel, copper, ruthenium, rhodium, palladium, iridium and platinum dispersed on the support. Most preferably the catalyst comprises ruthenium dispersed on the support. If the metal is one or more noble metals (e.g. ruthenium, rhodium, palladium, iridium or platinum), then the amount of metal is suitably from 0.05 to 5wt%, based on the weight of the metal oxide support, preferably from 0.1 to 2wt%. If the metal is one or more base metals (e.g. iron, cobalt, nickel, copper), then the amount of metal is suitably from 1 to 80wt%, based on the weight of the metal oxide support, preferably from 2 to 50wt%, more preferably from 5 to 20wt%.

The oxide support is a mixed metal oxide comprising at least 20wt% titania and at least 20wt% zirconia (based upon the weight of the oxide support). Preferably the metal oxide comprises at least 40wt% titania and 40wt% zirconia.

At least 80wt% of the mixed metal oxide is made up of titania and zirconia. It is preferred that at least 80wt% of the zirconia is in the monoclinic phase (based upon the weight of the zirconia) but the titania can be in any phase (e.g. the monoclinic phase or the tetragonal phase). In a particular embodiment, the oxide support is a mixed metal oxide consisting only of titania and zirconia. In another embodiment, there can be up to 20wt% of another metal oxide which is preferably chosen from oxides of silicon, yttrium and cerium.

In an embodiment of the invention the oxide support has been subjected to a heat treatment, preferably before the catalytic metal is deposited onto the oxide support. Suitably the metal oxide support is heated in liquid water to a temperature of at least 150°C for a period of at least 2 hours. Preferably the metal oxide support is heated to a temperature of at least 200°C. The metal oxide support is suitably heated to a temperature of less than 350°C, preferably less than 300°C and more preferably less than 250°C.

The process for the hydrogenation or hydrogenolysis of an oxygenate takes place in a reactor. The temperature in the reactor is suitably at least 80°C, preferably at least 130°C, more preferably at least 160°C, most preferably at least 190°C. The temperature in the reactor is suitably at most 300°C, preferably at most 280°C, more preferably at most 250°C, most preferably at most 230°C. Preferably, the reactor is heated to a temperature within these limits before addition of any starting material and is maintained at such a temperature as the reaction proceeds. Operating at higher temperatures has the potential disadvantage of increased amounts of side-reactions, leading to lower yield.

The pH in the reactor is in the range of from 2.5 to 10, preferably from 3 to 7 and most preferably from 3.5 to 5. The preferred pH is suitably maintained by using a buffer. Suitable buffers will be known to the skilled person but include sodium acetate. The amount of buffer supplied to the reactor is suitably from 0.01 to 10wt% of buffer based on the total weight of feedstock supplied to the reactor, preferably from 0.1 to 1wt%. The preferred pH is a balance between reducing the amount of side reactions and maximising the yield (the inventors' investigations suggest that higher pH gives fewer side reactions but lower pH gives better catalyst activity).

The pressure in the reactor is suitably at least 1 MPa, preferably at least 2 MPa, more preferably at least 3 MPa. The pressure in the reactor is suitably at most 25 MPa, preferably at most 20 MPa, more preferably at most 18 MPa. Preferably, the reactor is pressurised to a pressure within these limits by addition of hydrogen before addition of any reactant or water and is maintained at such a pressure as the reaction proceeds through on-going addition of hydrogen.

A second catalyst is preferably present in the reactor. The second active catalyst preferably comprises one or more homogeneous catalysts selected from tungsten or molybdenum, or compounds or complexes thereof. Most preferably, the second catalyst comprises one or more material selected from the list consisting of tungstic acid, molybdic acid, ammonium tungstate, ammonium metatungstate, ammonium paratungstate, tungstate compounds comprising at least one Group I or II element, metatungstate compounds comprising at least one Group I or II element, paratungstate compounds comprising at least one Group I or II element, heteropoly compounds of tungsten, heteropoly compounds of molybdenum, tungsten oxides, molybdenum oxides and combinations thereof. This catalyst is a retro-aldol catalyst, and in a preferred embodiment of the invention, the retro-aldol reaction and hydrogenation/hydrogenolysis take place in the same reactor. In other embodiments of the invention, a retro-aldol reaction may occur in a separate reactor prior to the hydrogenation/hydrogenolysis.

The residence time in the reactor is suitably at least 1 minute, preferably at least 2 minutes, more preferably at least 5 minutes. Suitably the residence time in the reactor is no more than 5 hours, preferably no more than 2 hours, more preferably no more than 1 hour.

The present invention is further illustrated in the following Examples.

### Procedure for testing hydrothermal stability of support materials

The experiments were conducted in 250ml Berghoff autoclaves with 200ml inserts, which were filled with 150ml of water. The pH of the water was adjusted to 3 by addition of acetic acid. The minimum amount of material used per test was 2g.

The water was heated to 250°C by placing the autoclaves in an oven. Under those conditions, an autogenous pressure of ~40 bar was obtained in the autoclave. After the experiment, the catalyst support materials were separated from the water phase by cold filtration.

### Analysis of catalyst support materials

The fresh and treated (filter cake) catalyst material were analysed by x-ray diffraction to determine the phase composition and the crystallite size. Occasionally also BET analyses were conducted. Leaching of support components was evaluated for some samples by hydrothermally treating 15g of catalyst in 150ml of water for 15 hrs (250°C, pH=3). The effluent was separated from the solid material by filtration. Both solids and filtration effluent were analysed for support components by ICP (Inductively coupled plasma).

### Support 1: SiO₂-doped monoclinic-ZrO₂ (not according to the invention)

The hydrothermal stability of SiO₂-doped monoclinic-ZrO₂ was assessed as described above (T=250°C, exposure time = 70hrs, pH=3). The patterns of the untreated and treated material were virtually identical, indicating that there is no significant difference in crystalline composition. All reflections could be assigned to zirconia modifications. Strong signals originated from the monoclinic phase and weaker signals could be attributed to either the tetragonal or cubic modifications. Strong overlap due to reflection broadening prevented an unambiguous discrimination between these last two phases. Rietveld refinement found a monoclinic : (tetragonal/cubic) ratio of 96:4 for both samples. The average crystallite size of the monoclinic phase was approximately 17 nm for both samples.

Leaching of the support components (Si, Zr) was studied as described above. The filtrate solution remained cloudy. Resting the cloudy solution resulted in settlement of the dust (solid fraction). The solid, clear solution and cloudy solution were analysed by ICP to determine the amounts of Si and Zr in the fractions. The Si/Zr mass ratio of the solid is in the range of 0.08 - 0.12. The clear solution contained mainly Si and hardly any Zr (Si/Zr mass ratio > 12), indicating that Si is likely leaching selectively from the material. The quantitative amount was very low as only approximately 0.1mass% of the silica leached from the solid material into the solution.

This example shows that SiO₂-doped m-ZrO₂ is relatively stable when subjected for 70 hrs at test conditions, and is therefore an interesting catalyst support for catalytic applications in aqueous phase.

### Support 2: SiO₂-doped tetragonal-ZrO₂ (not according to the invention)

The hydrothermal stability of SiO₂-doped tetragonal-ZrO₂ (St.Gobain-NorPro, SZ61152) was assessed as described above (T=250°C, exposure time =70 hrs, pH=3). The diffraction patterns of untreated and treated SiO₂-doped t-ZrO₂ were similar, but displayed some significant differences. All reflections in the patterns could be attributed to zirconia modifications. The strongest signals originated from the tetragonal modification. There could also be a small amount of the cubic modification present; strong overlap with reflections from the tetragonal modification due to reflection broadening prevented an unambiguous identification. Minor reflections could be attributed to the monoclinic modification. Rietveld refinement found a (tetragonal/cubic) : monoclinic ratio of approximately 90:10 before treatment and 80:20 after treatment. The average crystallite size of the tetragonal zirconia modification was approximately 14nm in both samples. The reflection intensity of the monoclinic zirconia modification was too low for a reliable crystallite size determination.

This example shows that SiO₂-doped t-ZrO₂ is relatively stable when subjected for 70 hrs to test conditions, and is therefore an interesting catalyst support for catalytic applications in aqueous phase.

### Support 3: CeO₂-doped tetragonal-ZrO₂ (not according to the invention)

The hydrothermal stability of a CeO₂-doped tetragonal-ZrO₂ (St.Gobain-NorPro, SZ61191) was assessed as described above (T=250°C, exposure time = 70 hrs, pH=3). The diffraction patterns of untreated and treated material were very similar. The observed peak positions were in very good fit with the reference patterns of the tetragonal modification of cerium doped zirconia, Zri-*ₓ*Ce*ₓ*O₂, especially for those patterns with x in the range 0.1-0.2. This phase has a structure that is isomorphous with the tetragonal modification of pure ZrO₂, but has a slightly larger unit cell, corresponding to the observed shift of the reflections. The diffraction patterns showed a weak, broad feature that could indicate the presence of an amorphous or pseudo-crystalline phase. In the pattern of the treated material, weak peaks were visible that correspond to the monoclinic modification of ZrO₂.

Based on RIR-values, the concentration of monoclinic zirconia modification in the treated material was estimated to be in the 5-10% range.

The average crystallite of the cerium-doped zirconia appeared to have slightly increased from 13 to 17 nm upon treatment.

This example shows that CeO₂-doped t-ZrO₂ is relatively stable when subjected for 70 hrs to test conditions, and is therefore an interesting catalyst support for catalytic applications in aqueous phase.

### Support 4: Y₂O₃-doped tetragonal-ZrO₂ (not according to the invention)

The hydrothermal stability of 8wt% Y₂O₃-doped tetragonal-ZrO₂ (St.Gobain-NorPro, SZ61157) was assessed as described above (T=250°C, exposure time = 15 hrs, 70 hrs and 18 days, pH=3). The patterns of the untreated and 18 days treated material showed a good resemblance to the reference patterns of (Y,Zr)O₂ mixed oxides that are isomorphous with the tetragonal ZrO₂ polymorph. The differences in reflection width showed that the average crystallite size increased from approximately 13 to approximately 16nm after 18 days (17nm after 70 hrs). The BET surface area of the fresh materials was 120m²/g and decreased to 61m²/g after 18 days treatment (75m²/g after 15 hrs treatment).

Leaching of the support components (Y,Zr) was studied. The filtrate solution remained cloudy. Resting the cloudy solution resulted in settlement of the dust. The solid, clear solution and cloudy solution were analysed by ICP to determine the amounts of Zr and Y in the fractions. The Y/Zr mass ratio in the solid is in the range of 0.10 - 0.16. The clear solution contains mainly Y and hardly any Zr (Y/Zr mass ratio > 122), indicating that Y is likely leaching from the material. The quantitative amount is significant as approximately ~1wt% of the yttrium leached from the solid material into the solution.

This example shows that Y₂O₃-doped tetragonal-ZrO₂ undergoes particle size growth and a resulting decrease in BET surface area in the initial stage of the treatment. However, the material seems to have stabilized after 70 hrs. The surface area decreased as results of the initial transformations but the surface areas of the stabilized materials are still relatively high (~70m²/g) and therefore this might is an interesting catalyst support for catalytic applications in aqueous phase.

### Support 5: ZrO₂-TiO₂

The hydrothermal stability of ZrO₂-TiO₂ (St.Gobain-NorPro, ST31140) was assessed as described above (T=250°C, exposure time = 70 hrs and 18 days, pH=3). The patterns of the untreated and 70 hrs days treated material showed the same set of reflections, albeit with different relative intensities. The observed reflections could be assigned to the monoclinic and tetragonal zirconia modifications and to tetragonal titania. The mixed zirconium-titanium oxide (Ti,Zr)O₂ is an isomorph of anatase and can therefore not be distinguished from that phase at the resolution of the current patterns. Rietveld refinement was used to quantify the relative concentration of the phases present (Table 1):

**Table 1**

| | untreated | 70 hrs treatment | 18 days treatment |
|---|---|---|---|
| Phase | relative conc. %(m/m) | | |
| monoclinic ZrO₂ | 50 | 53 | 56 |
| tetragonal ZrO₂ | 12 | 8 | 5 |
| tetragonal TiO₂ | 38 | 39 | 39 |
| | (m²/g) | | |
| BET | 93 | 72 | 68 |

The tetragonal ZrO₂ tends to transform to the monoclinic phase during treatment. The tetragonal phase of TiO₂ in this material was found to be stable under these conditions.

Overlap hinders an accurate determination of the average crystallite sizes. However, careful inspection and comparison of the diffraction patterns of the fresh material makes it clear that the average crystallite size of tetragonal titania (~40nm) is larger than that of monoclinic zirconia (~20nm). For both phases the reflection width decreases upon treatment, indicating an increase in average crystallite size of at least 5nm after 18 days treatment. The growth in crystallite size is probably also the underlying cause for the observed decrease in BET surface area from 93 to 68m²/g after 18 days treatment (72m² after 15 hrs).

ZrO₂-TiO₂ undergoes structure transformations during the beginning of the hydrothermal treatment. However, the material seems to have stabilized after 70 hrs. The surface area decreased as results of the initial transformations but the surface areas of the stabilized materials is still relatively high (~70m²/g) and therefore this might be an interesting catalyst support with relatively high surface area for catalytic applications in aqueous phase.

### Activity of Hydrogenation Catalysts with stable supports

The hydrogenation activity of catalysts was tested in a process for the hydrogenation of glycolaldehyde to ethylene glycol. 30g of water and 0.3g of glycolaldehyde and hydrogen (101 bar) were fed to the catalyst. The reactants were subjected to stirring at 1450rpm and a temperature of 195°C for 75 minutes.

Table 2 shows the different catalysts that were tested and table 3 shows the results of the hydrogenation reaction:

**Table 2**

| | Catalyst | Amount (g) | Heat treatment |
|---|---|---|---|
| Comparative Example 1 | 1% Ruthenium on SiO₂ | 0.045 | None |
| Comparative Example 2 | Raney Ni 2800 | 0.012 | None |
| Comparative Example 3 | Raney Co 2724 Ni Cr promoted | 0.015 | None |
| Example 1* | 0.4% Ru on Si-doped ZrO₂ | 0.113 | Support was treated for 70 hours in hot water (250°C, pH 3) |
| Example 2* | 0.3% Ru on Y-doped ZrO₂ | 0.045 | Support was treated for 70 hours in hot water (250°C, pH 3) |
| Example 3* | 0.3% Ru on Y-doped ZrO₂ | 0.15 | Support was treated for 70 hours in hot water (250°C, pH 3) |
| Example 4 | 0.4% Ru on TiO₂-ZrO₂ | 0.113 | Support was treated for 70 hours in hot water (250°C, pH 3) |

| | | | |
|---|---|---|---|
| * - not according to the invention | | | |

**Table 3**

| | Ethylene Glycol (wt%) | Propylene Glycol (wt%) | HA (wt%) | 1,2-BDL (wt%) | 1H₂BO (wt%) |
|---|---|---|---|---|---|
| Comparative Example 1 | 84.4 | 0.0 | 0.0 | 0.0 | 0.0 |
| Comparative Example 2 | 74.6 | 0.0 | 0.8 | 2.8 | 4.1 |
| Comparative Example 3 | 100.3 | 0.0 | 0.0 | 0.0 | 0.0 |
| Example 1* | 82.0 | 0.0 | 0.0 | 4.1 | 0.0 |
| Example 2* | 32.9 | 0.0 | 1.7 | 2.7 | 7.1 |
| Example 3* | 59.3 | 5.1 | 0.0 | 9.2 | 0.9 |
| Example 4 | 87.8 | 0.0 | 0.0 | 4.8 | 0.0 |

| | | | | | |
|---|---|---|---|---|---|
| * - not according to the invention | | | | | |

The examples show that good hydrogenation activity can be achieved with the catalysts that have thermally stable supports.

## Claims

1. A process for the hydrogenation or hydrogenolysis of an oxygenate in a reactor in the presence of a catalyst, hydrogen and liquid water,
wherein the catalyst comprises one or more elements from Groups 8 to 11 of the periodic table dispersed on an oxide support, and
wherein the oxide support is a mixed metal oxide comprising at least 20wt% titania and at least 20wt% zirconia and at least 80wt% of the mixed metal oxide is made up of titania and zirconia.

2. A process according to claim 1, wherein the oxygenate is present in or is derived from a saccharide-containing feedstock, and the process is a hydrogenation/hydrogenolysis reaction that produces glycols.

3. A process according to claim 1 or claim 2, wherein the catalyst comprises one or more elements from the group consisting of iron, cobalt, nickel, ruthenium, rhodium, palladium, iridium and platinum dispersed on the oxide support.

4. A process according to claim 3, wherein the catalyst comprises ruthenium dispersed on the oxide support.

5. A process according to any one of claims 1 to 4, wherein the oxide support is a mixed metal oxide comprising at least 20wt% titania and at least 20wt% zirconia, and wherein at least 80wt% of the zirconia is in the monoclinic phase.

6. A process according to any preceding claim, wherein the oxide support has been subjected to a heat treatment wherein the metal oxide support is heated in liquid water to a temperature of at least 150°C for a period of at least 2 hours.

7. A process according to any preceding claim, wherein a second catalyst is present in the reactor and the second active catalyst comprises one or more homogeneous catalysts selected from tungsten or molybdenum, or compounds or complexes thereof.

## Patentansprüche

1. Vorgang für die Hydrierung oder Hydrogenolyse eines Oxygenats in einem Reaktor in der Gegenwart von einem Katalysator, Wasserstoff und flüssigem Wasser, wobei der Katalysator ein oder mehrere Elemente aus den Gruppen 8 bis 11 des Periodensystems, dispergiert auf einem Oxidträger, umfasst, und
wobei der Oxidträger ein gemischtes Metalloxid ist, das wenigstens 20 Gew.-% Titanoxid und wenigstens 20 Gew.-% Zirkonoxid umfasst, und wenigstens 80 Gew.-% des gemischten Metalloxids aus Titanoxid und Zirkonoxid bestehen.

2. Vorgang nach Anspruch 1, wobei das Oxygenat in einem saccharidhaltigen Ausgangsmaterial vorhanden ist oder davon abgeleitet ist und der Vorgang eine Hydrierungs-/Hydrogenolysereaktion ist, die Glykole erzeugt.

3. Vorgang nach Anspruch 1 oder 2, wobei der Katalysator ein oder mehrere Elemente aus der Gruppe umfasst, die aus Eisen, Kobalt, Nickel, Ruthenium, Rhodium, Palladium, Iridium und Platin, dispergiert auf dem Oxidträger, besteht.

4. Vorgang nach Anspruch 3, wobei der Katalysator Ruthenium, dispergiert auf dem Oxidträger, umfasst.

5. Vorgang nach einem der Ansprüche 1 bis 4, wobei der Oxidträger ein gemischtes Metalloxid ist, das wenigstens 20 Gew.-% Titanoxid und wenigstens 20 Gew.-% Zirkonoxid umfasst, und wobei wenigstens 80 Gew.-% des Zirkonoxids in der monoklinen Phase sind.

6. Vorgang nach einem der vorhergehenden Ansprüche, wobei der Oxidträger einer Wärmebehandlung unterzogen wurde, wobei der Metalloxidträger in flüssigem Wasser auf eine Temperatur von wenigstens 150 °C für einen Zeitraum von wenigstens 2 Stunden erwärmt wird.

7. Vorgang nach einem der vorhergehenden Ansprüche, wobei ein zweiter Katalysator in dem Reaktor vorhanden ist und der zweite aktive Katalysator einen oder mehrere homogene Katalysatoren umfasst, die aus Wolfram oder Molybdän oder Verbindungen oder Komplexen davon ausgewählt sind.

## Revendications

1. Procédé d'hydrogénation ou d'hydrogénolyse d'un composé oxygéné dans un réacteur en présence d'un catalyseur, d'hydrogène et d'eau liquide,
dans lequel le catalyseur comprend un ou plusieurs éléments des groupes 8 à 11 du tableau périodique dispersés sur un support d'oxyde, et dans lequel le support d'oxyde est un oxyde métallique mixte comprenant au moins 20 % en poids d'oxyde de titane et au moins 20 % en poids de zircone et au moins 80 % en poids de l'oxyde métallique mixte est composé d'oxyde de titane et de zircone.

2. Procédé selon la revendication 1, dans lequel le composé oxygéné est présent dans ou est dérivé d'une charge d'alimentation contenant des saccharides, et le procédé est une réaction d'hydrogénation/hydrogénolyse qui produit des glycols.

3. Procédé selon la revendication 1 ou la revendication 2, dans lequel le catalyseur comprend un ou plusieurs éléments du groupe consistant en fer, cobalt, nickel, ruthénium, rhodium, palladium, iridium et platine dispersés sur le support d'oxyde.

4. Procédé selon la revendication 3, dans lequel le catalyseur comprend du ruthénium dispersé sur le support d'oxyde.

5. Procédé selon l'une quelconque des revendications 1 à 4, dans lequel le support d'oxyde est un oxyde métallique mixte comprenant au moins 20 % en poids d'oxyde de titane et au moins 20 % en poids de zircone, et dans lequel au moins 80 % en poids de la zircone est dans la phase monoclinique.

6. Procédé selon l'une quelconque des revendications précédentes, dans lequel le support d'oxyde a été soumis à un traitement thermique dans lequel le support d'oxyde métallique est chauffé dans de l'eau liquide à une température d'au moins 150 °C pendant une durée d'au moins 2 heures.

7. Procédé selon l'une quelconque des revendications précédentes, dans lequel un second catalyseur est présent dans le réacteur et le second catalyseur actif comprend un ou plusieurs catalyseurs homogènes choisis parmi le tungstène ou le molybdène, ou leurs composés ou complexes.
